# EUROPEAN PATENT APPLICATION

(11) **EP 2 902 043 A1**
(43) Date of publication of application: **05.08.2015**
(21) Application number: 13841884.3
(22) Date of filing: 28.08.2013
(51) Int. Cl.: A61L 27/28, A61L 27/54, A61L 27/56

(54) **MULTIFUNCTIONAL COMPOSITE DRUG COATING SUSTAINED RELEASE SYSTEM AND METHOD FOR MANUFACTURING SAME**

(30) Priority: 28.09.2012 CN 201210376203
(71) Applicant: Shanghai Microport Orthopedics Co., Ltd, Pudong New District, Shanghai 201318 (CN)
(72) Inventor: GENG, Fang, Shanghai 201318 (CN); PAN, Licun, Shanghai 201318 (CN); ZHANG, Jun, Shanghai 201318 (CN); WEN, Xiaoyi, Shanghai 201318 (CN); LIN, Zhong, Shanghai 201318 (CN)
(74) Representative: Kling, Simone
(86) International application number: PCT/CN2013/082459
(87) International publication number: WO 2014/048208

(57) **Abstract**

A multifunctional composite drug coating sustained release system includes a transition layer and a drug-loaded degradable coating, wherein the transition layer is a ceramic transition layer having different porosities and the transition layer includes a dense lower layer and a porous upper layer. The multifunctional composite drug coating sustained release system is helpful to internal fixation and also has an antibacterial efficacy. A method for manufacturing the multifunctional composite drug coating sustained release system is also disclosed which includes: preparing a biocompatible ceramic transition layer on a metal surface; and then preparing a drug-loaded degradable coating on a surface of the biocompatible ceramic transition layer.

## Description

### TECHNICAL FIELD

The present invention relates to medical appliances and, in particular, to titanium or magnesium alloy orthopedic implants. More particularly, the invention is directed to a multifunctional composite drug coating sustained release system and methods for manufacturing the same.

### BACKGROUND

In clinical orthopedics, infection is one of the complications associated with internal fixation operations, which can cause internal fixation failure or the formation of highly drug-resistant biofilms. In the latter case, even systemic application of a large dose of antibiotics could not achieve an effective antibiotic concentration in local lesion tissue, thus rendering the infection long-lasting and refractory, which finally necessitates the removal of the deployed implant. Other possible consequences of post-operative infection include, for example, prolonged hospitalization for systemic antimicrobial therapy, multiple surgical debridement revisions, treatment regimen changes, and limb amputation or even mortality. For instances, half of the two million nosocomial infection cases recorded yearly in the United States are implant-related infections which cause $11 billion of losses, and in the United Kingdom, implant-related infections result in a total loss of 7-11 million pounds every year. In the United States, inpatient expenditure for orthopedic patients with post-operative infection is 518% of that for uninfected patients.

Conventionally, many methods have been taught for reducing the occurrence of post-operative infection. One of the methods is to mix antibiotic drugs in bone cement.

Patent Application No. 200810060270.6 discloses a method for improving local drug release from antibiotic-loaded bone cement. In the method, acrylic bone cement loaded with antibiotics is submerged in phosphate buffer solution in a flask and a micro-bubble agent is then injected therein. After an ultrasonic probe is attached to the bottom of the flask, an associated ultrasonic generator is activated to produce ultrasonic waves with a frequency of 20-100 kHz and a spatial-average temporal-average intensity ranging from 20 MW/cm² to 3 W/cm². The ultrasonic generator may either operate in a continuous mode, or in a pulsed mode at a duty ratio of 20-100% and a modulation pulse frequency of 1 Hz to 10 kHz. This invention can achieve a larger total amount of antibiotic-loaded bone cement based drug release occurring at an increased speed and lasting for a certain period of time. Accordingly, it is capable of effectively preventing the formation of biofilms, increasing microbial susceptibility to antibiotics and effectively inhibiting bacterial adhesion and biofilm accumulation, thereby improving antimicrobial efficacy of antibiotic-loaded bone cement and expanding its scope of application.

Patent Application No. 200810036627.7 discloses an osteoinductive degradable carrier system for orthopedic drugs and its preparation methods. The drug carrier system essentially consists of a cured product of a bioactive glass serving as a carrier material and a therapeutic drug carried thereby. The carrier material can be obtained by reacting a highly reactive boron-containing bioactive glass with a curing buffer and further subjecting it to a rapid curing process such that the glass has a desired strength. A variety of drugs, helpful in bone disease treatment and with their thermal stability not subject to any limitations, including antibiotics, bone growth factors, anti-tuberculosis drugs and antineoplastic drugs, are loadable in the carrier material to form an integral drug carrier system spontaneously degradable in vivo under the action of tissue fluids to transform to a porous structure which provides for diffusion paths for sustained drug release and thus achieves prolonged uniform drug release. The drug carrier system is made usable in the treatment of a wide range of orthopedic diseases by its high biocompatibility, bioactivity and osteoinductivity.

Patent Application No. 200820158054.0 discloses an antimicrobial joint prosthesis featuring a bone-surface contacting portion consisting of a matrix and a 5-350 µm thick overlying silver-containing coating. The coating over the joint prosthesis according to this utility model can be implemented as either a silver coating or a silver/hydroxyapatite coating, which is capable of sustained release of silver ions that can kill bacteria and free from the killed bacteria for repeated further sterilizations. This not only makes the novel prosthesis a greatly reduced risk of causing post-implantation complications such as infection and loosening, but also circumvents the issues associated with the use of conventional antibiotic-loaded bone cement. Further, the prosthesis also possesses excellent biocompatibility and bioactivity.

In addition to the above described "bone cement mix-in" approach, antibiotic drugs have also been conventionally incorporated in coatings. Patent Application No. 200510131656.8 is related to an antibiotic coating for use in implants. The coating features its composition including a mixture of at least one saturated organic, hydrophobic, low-molecular-weight matrix having a melting point of 45-100 °C and a low-molecular-weight hydrophobic additive solubilized in the matrix material, and an antibiotic suspended in the mixture. In other words, the coating is made of the matrix/additive mixture dissolved therein with an antibiotic that is suitable to be mixed with the mixture.

With regard to titanium and magnesium alloy implants, it has been taught conventionally to form bioactive coatings (including antimicrobial coatings) on their surfaces. Patent Application No. 200910006839.5 discloses a method of preparing a composite coating consisting of a hydroxyapatite layer and a surface active titanium-based layer. Patent Application No. 200710157568.4 discloses a method of preparing a hydroxyapatite/polylactide composite bio-coating on the surface of a magnesium alloy article. Patent Application No. 200910072105.7 discloses a method of forming a silver-loaded bioactive antibacterial coating on the surface of a magnesium or titanium alloy article by surface ultrasonic waves used in combination with micro-arc oxidation. This method can be used to produce a bio-coating consisting of a dense lower layer and a porous upper layer and containing calcium, phosphorus and silver elements which can improve the bioactivity and corrosion resistance of magnesium and titanium and thus reduce the occurrence of bacterial infection caused by the alloy article when it is used as an implant.

However, the conventional technologies, including those to coat antibiotics on an implant, form a single-layered polymeric coating on the surface of an alloy article, load drugs in a single-layered ceramic coating or nanoceramic particles, and fabricate a three-dimensional bone tissue engineered scaffold from ceramics and polymeric materials, all suffer from their own deficiencies. For example, drug-loaded single-layered polymeric coatings usually have low bonding strength which is prone to causing uncontrolled drug release; currently existing three-dimensional bone tissue engineered scaffolds made from both ceramics and polymeric materials are inferior to natural bone tissues in terms of mechanical strength and hence need further improvements; and single-layered ceramic coatings cannot load drugs enough to achieve a sustained release effect.

Therefore, there is still a need for improvement in drug release control and anti-infection performance of prosthetic implants including orthopedic implants and cardiovascular stents.

### SUMMARY OF THE INVENTION

In order to overcome the above described deficiencies of the conventional technologies, the present invention provides a porous, degradable system for sustained release of antibacterial drugs formed on the surface of a matrix, notable a titanium or magnesium alloy matrix. The system assumes the form of a multifunctional composite coating including a ceramic transition layer having different porosities and a degradable coating containing a drug for sustained release. The porous structure of the ceramic transition layer overlying the alloy matrix can provide the degradable coating with increased bonding strength while ensuring effective drug release. The system entails a unique and effective surface treatment for orthopedic implants which ensures both effective internal fixation and significant antibacterial efficacy.

Specifically, the present invention relates to a multifunctional composite drug coating sustained release system. The system is in the form of a multifunctional composite coating including a porous transition layer and a drug-containing gradable coating. According to the invention, the transition layer is a ceramic transition layer with different porosities, preferably a TiO₂ transition layer, Mg(OH)₂ transition layer or MgO transition layer. As used herein, the term "porosity" denotes a fraction of the volume of pores within a certain portion of the ceramic transition layer over the total volume of the portion. According to the invention, the porous transition layer may be disposed on the surface of an alloy matrix formed of titanium and/or magnesium. According to the invention, in case of an orthopedic implant, the drug may be one or a mixture of antibacterial drugs which are preferred to be antibiotics and the antibiotics are preferably one or more selected from gentamicin and vancomycin. In case of a cardiovascular stent, the drug may be one or more selected from a group consisting of anticancer drugs, anticoagulants, microbial immunosuppressive drugs and anti-restenosis drugs. According to the invention, the degradable coating may be a degradable polymeric material, with one or more selected from polylactic acid (PLA), poly lactic-co-glycolic acid (PLGA) and collagen being preferred. According to the invention, the porous ceramic transition layer includes a dense lower layer and a porous upper layer which may have a pore diameter ranging from 100 nm to 3 µm, and the transition layer may have a thickness of 10-50 µm. According to the invention, the drug-containing coating may generally have a thickness of 2-10 µm and the thickness may be adjustable depending on the practical need for the amount of the drug.

The multifunctional composite drug coating sustained release system according to the present invention may be used either in an orthopedic implant or in a cardiovascular stent such as, for example, a magnesium alloy stent.

The present invention also relates to a method of preparing a multifunctional composite drug coating sustained release system. The method includes: forming a biocompatible ceramic transition layer on the surface of a metal matrix; and then forming a degradable drug-loaded coating on the surface of the biocompatible ceramic transition layer.

The degradable polymeric material according to the present invention may be selected from, but not limited to, degradable polymeric materials such as L-polylactic acid (L-PLA), DL-polylactic acid (DL-PLA), polyglycolic acid (PGA), poly-ε-caprolactone (PCL), poly(trimethylene carbonate) (PTMC), poly(p-dioxanone) (PPDO), amino acid-derived polycarbonates (PDTE) and polyorthoesters (POE); and blends of any two of the above degradable polymeric materials, including, but not limited to, L-PLA/PCL and DL-PLA/PCL blends. In addition, the degradable polymeric material may also be selected from degradable carrier materials including: degradable natural materials, such as collagen, chitosan, gelatin, cellulose and silk fibroin; and their mixtures with the degradable polymeric materials.

The drug according to the present invention may be selected from different groups depending on the practical needs. For example, for the purpose of microbial inhibition and prevention of orthopedic implant infection, the drug may be selected from antibiotic drugs, including, but not limited to: 1) β-lactams, including penicillins and cephalosporins, such as thienamycins, monobactams, β-lactamade inhibitors and methoxypeniciuins; 2) aminoglycosides, including streptomycin, gentamicin, kanamycin, tobramycin, amikacin, neomycin, ribostamycin, micronomicin and astromicin; 3) tetracyclines, including tetracycline, oxytetracycline, chlortetracycline and doxycycline; 4) chloramphenicols, including chloramphenicol and thiamphenicol; 5) macrolides, including erythromycin, albomycin, erythromycin estolate, erythromycin ethylsuccinate, azithromycin, acetylspiramycin, midecamycin and josamycin; 6) other antibiotics acting on Gram-positive bacteria, such as lincomycin, clindamycin, vancomycin and bacitracin; 7) other antibiotics acting on Gram bacteria, such as polymyxin, fosfomycin, ciramycin, cycloserine and rifampicin; 8) antifungal antibiotics, such as griseofulvin; 9) anticancer antibiotics, such as mitomycin, actinomycin D, bleomycin and Adriamycin; and 10) immunosuppressive antibiotics, such as cyclosporine, with gentamicin and vancomycin being preferred.

For example, in order to prevent neointimal hyperplasia after a cardiovascular stent is implanted, the drug according to the present invention may be one or more selected from anticancer drugs, anticoagulants, microbial immunosuppressive drugs and anti-restenosis drugs. The anticancer drugs may be one or more selected from methotrexate, purines, pyrimidines, plant alkaloids, epothilones, triptolide compounds, antibiotics (notably actinomycin D), hormones and antibodies. From among the plant alkaloids, mention may notably be made of paclitaxel. The anticoagulants may be one or more selected from heparin, aspirin, hirudin, colchicine and platelet GPIIb/IIIa receptor antagonists. The platelet GPIIb/IIIa receptor antagonists may be one or more selected from tirofiban, abciximab and eptifibatide. The microbial immunosuppressive drugs may be one or more selected from cyclosporin A, tacrolimus and its analogues, despergualin, mycophenolate esters, rapamycin and its derivatives, FR-900520 substance from Streptomyces strains, FR-900523 substance from Streptomyces strains, daclizumab, pentanamide, kanglemycin C, spergualin, prodigiosin-25C, tranilast, myriocin, cyclosporin C, bredinin, mycophenolic acid, brefeldin A and ketosteroids. The anti-restenosis drugs may be one or more selected from batimastat, metalloproteinase inhibitors, 17β-estradiol, NO donors, 2-chlorodeoxyadenosine, 2-deoxycoformycin, fingolimod, mycophenolate sodium, ISA_{TX}247 (a cyclosporin A derivative), elsibucol, daclizumab, basiliximab, anti-thymocyte globulin, everolimus, methotrexate, neoral, cyclophosphamide, brequinar sodium, leflunomide and mizoribine.

After complete degradation of the multifunctional composite drug coating sustained release system according to the present invention, the ceramic transition layer on the matrix surface will further exhibit a high biocompatibility. In addition to metal oxides such as, for example, TiO₂, Mg(OH)₂ and MgO, materials that may be used to fabricate the biocompatible ceramic transition layer may further include calcium phosphate ceramics, silicate ceramics, carbonate ceramics, etc.

The present invention enables topical administration by forming on the surface of an implant a degradable system for sustained release of a drug, which can accurately deliver the drug to an intended location such that the drug can directly act on the lesion to be treated. This eliminates the reliance on blood for conveying the drug thereto and prevents ischemia of the lesion from affecting the drug's efficacy. The system can achieve effective control of drug release and a good antibacterial effect.

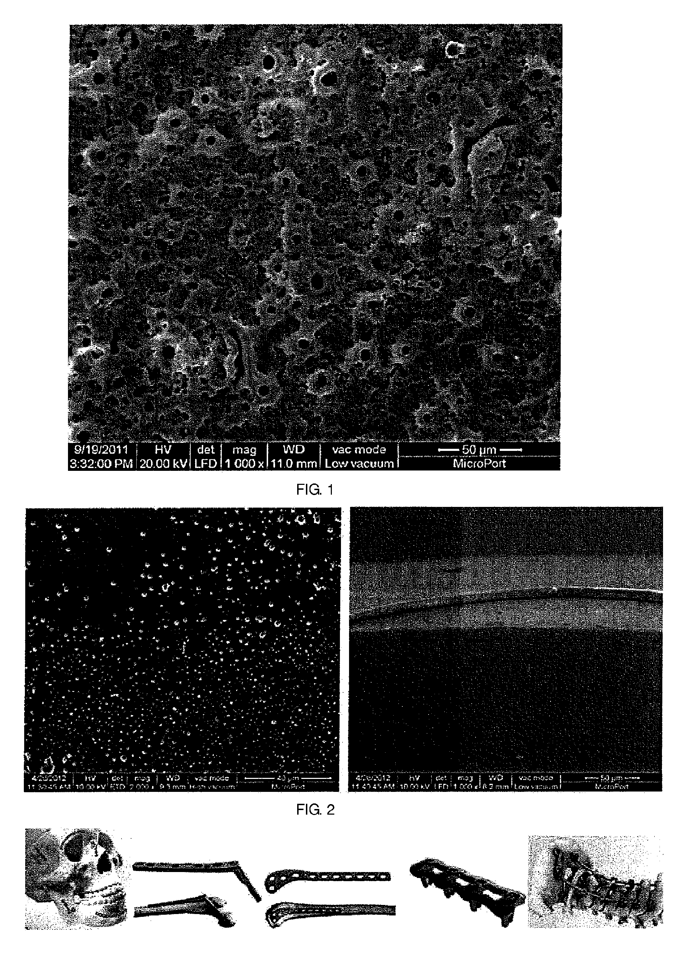

The porous degradable coating system for sustained drug release according the present invention are mainly deployed on a titanium or magnesium alloy surface of an orthopedic implant, for preventing or treating infection that is possibly associated with the implantation of the implant and thus facilitating its internal fixation. The deployment may be realized by first forming on the alloy surface a porous biocompatible ceramic transition layer (FIG. 1) and then forming on the surface of the transition layer a degradable drug-loaded coating (FIG. 2). The drug may be selected notably from antibiotics. The porous transition layer can provide not only increased bonding strength to the degradable drug-loaded coating but also a sustained release effect. The drug-loaded coating is a biodegradable material which can participate in normal metabolism in the body without exerting thereon toxic or side effects and can be finally excreted out of the body.

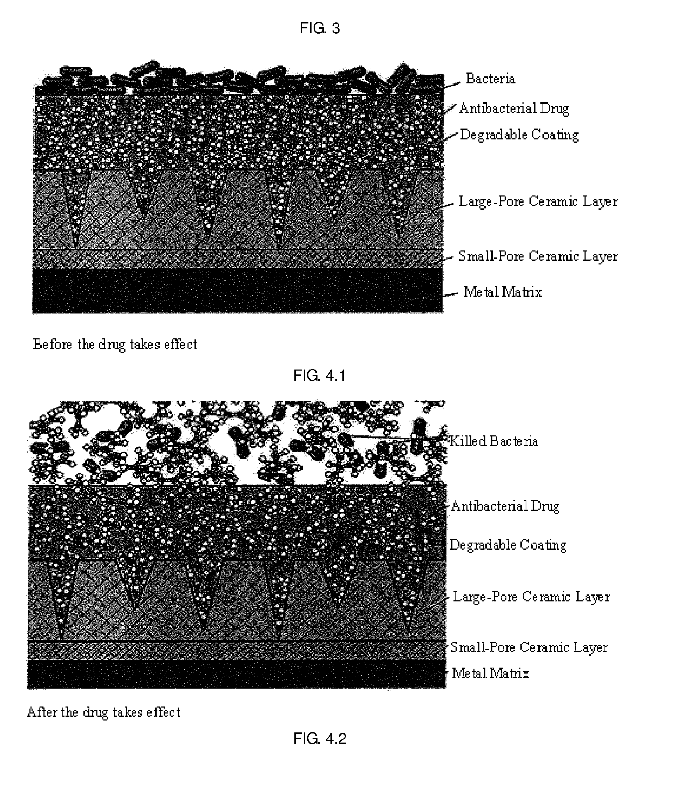

Orthopedic implants on which the system can be deployed include bone plates, bone screws, joints, spines and the like made of titanium and/or magnesium alloys, notably those prone to causing post-implantation infectious complications. The porous transition layer is fabricated from ceramics, while the drug-loaded coating may be made from a degradable polymeric material, a degradable ceramic, collagen, or a mixture of several of the above. The drug may be one or a mixture of different antibiotics. FIG. 3 shows several exemplary applicable orthopedic implants.

### BRIEF DESCRIPTION OF THE DRAWINGS

Features of the invention will become apparent from the following description, taken in conjunction with the accompanying drawings. It is apparent that what are set forth in the drawings are merely several specific embodiments described in this application. The invention therefore includes, but is not limited to, those set forth therein.
FIG.1 shows the porous profile of a transition layer coated over a metal surface.
FIG. 2 shows the surface and thickness of a drug-loaded coating.
FIG. 3 shows several types of applicable orthopedic implants.
FIG. 4 shows a multifunctional degradable system for sustained drug release before and after the drug takes effect.

### DETAILED DESCRIPTION

For a better understanding of the present invention, certain preferred features of the present invention are described in the following Examples. The description is merely illustrative of the features and advantages of magnesium or titanium alloy orthopedic implants in accordance with the invention rather than limiting the scope of the invention.

### EXAMPLE 1

A porous TiO₂ transition layer was formed on the surface of a titanium alloy orthopedic implant. The degradable polymeric material was selected as DL-PLA, and the drug was gentamicin.

### Formation steps:

1. Formation of Porous Ceramic Transition Layer:
   A porous ceramic transition layer having a dense TiO₂ lower layer and a porous TiO₂ upper layer with a pore diameter ranging from 100 nm to 3 µm and a thickness of 10-50 µm was formed over the surface of a titanium or magnesium alloy matrix by means of plasma oxidation process using a voltage increasing in steps from 100 V to 500 V, a current of 1-3 A, a process time of 1-20 minutes and an oxidation solution containing silicates, phosphates, etc.
2. Preparation of Drug Solution: Precisely weighed DL-polylactic acid (DL-PLA) (PDL04) was dissolved in tetrahydrofuran (THF) to form a polymeric solution which was then added with a pre-prepared aqueous solution of gentamicin, resulting in a mixture solution of DL-PTA and gentamicin. The resulting drug-containing solution was sprayed or dipped on the surface of the transition layer resulting from step 1 and was dried until there was no change in weight. A thickness of the drug-loaded coating is generally 2-10 µm.
3. Antimicrobial effect of the coating was evaluated by biological tests and the results showed that the drug release concentration was maintained for one month at a level higher than a minimal inhibitory concentration of 0.5 µg/ml, demonstrating good sustained-release and antibacterial effects of the coating.

### EXAMPLE 2

A porous TiO₂ transition layer was formed on the surface of a titanium alloy orthopedic implant. The degradable coating was made from a mixture of PLGA and collagen, and the drug was a mixture of gentamicin and vancomycin.

### Formation steps:

1. Formation of Porous Ceramic Transition Layer:
   A porous ceramic transition layer having a dense TiO₂ lower layer and a porous TiO₂ upper layer with a pore diameter ranging from 100 nm to 3 µm and a thickness of 10-50 µm was formed over the surface of the metal matrix by means of plasma oxidation process using a voltage increasing in steps from 100 V to 500 V, a current of 1-3 A, a process time of 1-20 minutes and an oxidation solution containing silicates, phosphates, etc.
2. Preparation of Drug Solution: Precisely weighed PLGA and collagen were dissolved in tetrahydrofuran (THF) to form a polymeric solution which was subsequently added with a pre-prepared aqueous solution of gentamicin and vancomycin, resulting in a mixture solution. The resulting drug-containing solution was sprayed or dipped on the surface of the transition layer resulting from step 1 and was dried until there was no change in weight. A thickness of the drug-loaded coating is generally 2-10 µm.
3. Antimicrobial effect of the coating was evaluated by biological tests and the results showed that the drug release concentration was maintained for one month at a level higher than the minimal inhibitory concentration of 0.5 µg/ml, demonstrating good sustained-release and antibacterial effects of the coating.

### EXAMPLE 3

A porous Mg(OH)₂/MgO transition layer was formed on the surface of a magnesium alloy orthopedic implant. The degradable polymeric material was selected as DL-PLA, and the drug was gentamicin.

### Formation steps:

1. Formation of Porous Ceramic Transition Layer:
   A porous ceramic transition layer having a dense MgO lower layer and a porous MgO/Mg(OH)₂ upper layer with a pore diameter ranging from 100 nm to 3 µm and a thickness of 5-30 µm was formed over the surface of the metal matrix by means of plasma oxidation process using a voltage increasing in steps from 20 V to 200 V, a current of 0.1-2 A, a process time of 1-20 minutes and an oxidation solution containing silicates, phosphates, etc.
2. Preparation of Drug Solution: Precisely weighed DL-PLA was dissolved in tetrahydrofuran (THF) to form a polymeric solution which was then added with a pre-prepared aqueous solution of gentamicin, resulting in a mixture solution of DL-PLA and gentamicin. The resulting drug-containing solution was sprayed or dipped on the surface of the transition layer resulting from step 1 and was dried until there was no change in weight. A thickness of the drug-loaded coating is generally 2-10 µm.
3. Antimicrobial effect of the coating was evaluated by biological tests and the results showed that the drug release concentration was maintained for one month at a level higher than the minimal inhibitory concentration of 0.5 µg/ml, demonstrating good sustained-release and antibacterial effects of the coating.

### EXAMPLE 4

A porous Mg(OH)₂/MgO transition layer was formed on the surface of a magnesium alloy orthopedic implant. The degradable coating was a mixture of PLGA and collagen and the drug was a mixture of gentamicin and vancomycin.

### Formation steps:

1. Formation of Porous Ceramic Transition Layer:
   A porous ceramic transition layer having a dense MgO lower layer and a porous MgO/Mg(OH)₂ upper layer with a pore diameter ranging from 100 nm to 3 µm and a thickness of 5-30 µm was formed over the surface of the metal matrix by means of plasma oxidation process using a voltage increasing in steps from 20 V to 200 V, a current of 0.1-2 A, a process time of 1-20 minutes and an oxidation solution containing silicates, phosphates, etc.
2. Preparation of Drug Solution: Precisely weighed PLGA and collagen were dissolved in tetrahydrofuran (THF) to form a polymeric solution which was subsequently added with a pre-prepared aqueous solution of gentamicin and vancomycin, resulting in a mixture solution. The resulting drug-containing solution was sprayed or dipped on the surface of the transition layer resulting from step 1 and was dried until there was no change in weight. A thickness of the drug-loaded coating is generally 2-10 µm.
3. Antimicrobial effect of the coating was evaluated by biological tests and the results showed that the drug release concentration was maintained for one month at a level higher than the minimal inhibitory concentration of 0.5 µg/ml, demonstrating good sustained-release and antibacterial effects of the coating.

FIGs. 4.1 and 4.2 show the multifunctional degradable drug sustained release system according to the present invention before and after the drug takes effect, respectively.

### Beneficial Effects of the Invention

The antibiotics-loaded porous degradable composite coating systems according to the present invention are mainly used in orthopedic implants. They can eliminate the existing risk of infection associated with implant internal fixation, in particular infection frequently occurring after such treatment for open fractures. The present invention entails a novel topical administration method in which a sustained antibiotics release system is formed on surface of the implant, which provides a controlled release rate to make the drug locally concentrate to achieve increased efficacy. In addition, as the drug is loaded in the carrier and is released with the degradation thereof, it is consumed by metabolism at a lower rate and thus has a prolonged treatment effect. Further, the carrier itself is a biodegradable material which can participate in normal metabolism in the body without exerting toxic or side effects and can be finally excreted out of the body.

The present invention can reduce post-implantation infection while not compromising the function of the implant. Further, the aforesaid topical administration method can address the low-efficiency and high-toxicity issues arising from the use of conventional modes of administration, thus avoiding the toxic and side effects attributed to systemic administration and the difficulty in delivering drug to lesion areas lacking blood supply.

The present invention is mainly used in orthopedic implants for internal fixation by forming on an orthopedic implant surface an antibiotics-loaded porous degradable composite coating system. A porous ceramic transition structure on the alloy matrix surface can provide the coating with an increased drug-loading capacity and an improved bonding strength with the degradable polymeric coating (i.e., an increased internal fixation capability). The antibiotics-loaded degradable polymeric coating has both sustained release and antibacterial effects. In addition, after its complete degradation, the ceramic structure overlying the implant surface can further exhibit a high biocompatibility. Therefore, the multifunctional coating system for sustained drug release according to the present invention can not only facilitate internal fixation but also has an antibacterial effect.

Description of the foregoing examples is presented merely for facilitating the understanding of the core principles of the present invention. It is noted that while many modifications and variations can be made by those having ordinary skill in the art without departing from the inventive concept disclosed herein, it is intended that the appended claims cover all such modifications and variations.

## Claims

1. A multifunctional composite drug coating sustained release system, comprising a transition layer and a degradable coating containing a drug, wherein the transition layer is a ceramic transition layer having different porosities, and further wherein the transition layer comprises a dense lower layer and a porous upper layer.

2. The multifunctional composite drug coating sustained release system of claim 1, wherein the ceramic transition layer is a TiO₂ transition layer, Mg(OH)₂ transition layer or MgO transition layer.

3. The multifunctional composite drug coating sustained release system of claim 1 or 2, wherein the porous upper layer has a pore diameter ranging from 100 nm to 3 µm.

4. The multifunctional composite drug coating sustained release system of claim 1, wherein the transition layer is disposed on a surface of an alloy matrix formed of titanium and/or magnesium.

5. The multifunctional composite drug coating sustained release system of claim 1, wherein in case of an orthopedic implant, the drug is one or a mixture of antibacterial drugs, and wherein in case of a cardiovascular stent, the drug is one or more selected from a group consisting of anticancer drugs, anticoagulants, microbial immunosuppressive drugs and anti-restenosis drugs.

6. The multifunctional composite drug coating sustained release system of claim 5, wherein the antibacterial drugs are antibiotics.

7. The multifunctional composite drug coating sustained release system of claim 6, wherein the antibiotics are one or both of gentamicin and vancomycin.

8. The multifunctional composite drug coating sustained release system of claim 1, wherein the degradable coating is a degradable polymeric material.

9. The multifunctional composite drug coating sustained release system of claim 8, wherein the degradable polymeric material is one or more selected from PLA, PLGA and collagen.

10. A method for preparing the multifunctional composite drug coating sustained release system as defined in claim 1, comprising: forming a biocompatible ceramic transition layer on a metal surface; and then forming a degradable drug-loaded coating on a surface of the biocompatible ceramic transition layer.

11. Use of the multifunctional composite drug coating sustained release system as defined in claim 1 in preparation of an orthopedic implant or a cardiovascular stent.
